(19) Europäisches Patentamt
European Patent Office
Office européen des brevets

(11) EP 4 352 072 B1

(12) EUROPEAN PATENT SPECIFICATION

(45) Date of publication and mention of the grant of the patent:
**04.02.2026 Bulletin 2026/06**

(21) Application number: **22820643.9**

(22) Date of filing: **13.06.2022**

(51) International Patent Classification (IPC):
***C07H 15/26*** *(2006.01)* ***C07D 475/08*** *(2006.01)*
***A61K 31/7052*** *(2006.01)* ***A61P 35/00*** *(2006.01)*
***C07H 1/00*** *(2006.01)*

(52) Cooperative Patent Classification (CPC):
**A61P 35/00; C07D 475/08; C07H 1/00; C07H 15/26**

(86) International application number:
**PCT/PL2022/050036**

(87) International publication number:
**WO 2022/260545 (15.12.2022 Gazette 2022/50)**

# (54) SYNTHESIS OF A NOVEL GLUCOSE-CONJUGATED METHOTREXATE AND ITS APPLICATION FOR THE TREATMENT AND PREVENTION OF NEOPLASMS

SYNTHESE EINES NEUEN GLUCOSEKONJUGIERTEN METHOTREXATS UND DESSEN ANWENDUNG ZUR BEHANDLUNG UND PRÄVENTION VON NEOPLASMEN

SYNTHÈSE D'UN NOUVEAU MÉTHOTREXATE CONJUGUÉ AU GLUCOSE ET SON APPLICATION POUR LE TRAITEMENT ET LA PRÉVENTION DE NÉOPLASMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2021 PL 43813421**

(43) Date of publication of application:
**17.04.2024 Bulletin 2024/16**

(73) Proprietor: **Uniwersytet Medyczny Im. Piastów Slaskich We Wroclawiu**
**50-367 Wroclaw (PL)**

(72) Inventors:
- **AGRAWAL, Siddarth**
  **55-010 Zerniki Wroclawskie (PL)**
- **WOZNIAK, Marta**
  **50-067 Wroclaw (PL)**
- **MAKUCH, Sebastian**
  **34-424 Skrzypne (PL)**
- **SZEJA, Wieslaw**
  **44-100 Gliwice (PL)**
- **PASTUCH-GAWOLEK, Gabriela**
  **44-121 Gliwice (PL)**
- **KRAWCZYK, Monika**
  **32-300 Olkusz (PL)**
- **WISNIEWSKI, Jerzy**
  **51-423 Wroclaw (PL)**
- **GAMIAN, Andrzej**
  **51-644 Wroclaw (PL)**
- **ZIÓLKOWSKI, Piotr**
  **52-430 Wroclaw (PL)**

(74) Representative: **Witek, Rafal**
**WTS Patent Attorneys**
**Witek, Sniezko & Partners**
**Ul. Rudolfa Weigla 12**
**53-114 Wroclaw (PL)**

(56) References cited:
**WO-A1-2013/127885 WO-A1-2022/260546**
**PL-A1- 426 731**

- **WOZNIAK MARTA ET AL: "In Vitro and In Vivo Efficacy of a Novel Glucose-Methotrexate Conjugate in Targeted Cancer Treatment", INTERNATIONAL JOURNAL OF MOLECULAR SCIENCES, vol. 22, no. 4, 9 February 2021 (2021-02-09), pages 1748, XP093075988, DOI: 10.3390/ijms22041748**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• ROSARIO PIGNATELLO ET AL.: "Lipophilic methotrexate conjugates with glucose-lipoamino acid moieties: Synthesis and in vitro antitumor activity", DRUG DEVELOPMENT RESEARCH, vol. 52, no. 3, March 2001 (2001-03-01), pages 454 - 461, XP055312801, Retrieved from the Internet <URL:https://doi.org/10.1002/ddr.1147> DOI: 10.1002/ddr.1147

## Description

Targeted prodrug design is currently widely used to reduce the adverse effects of cancer chemotherapy caused by the non-selective action of drugs on human tissues. In many studies evaluating the activity of anti-cancer drug analogues, active substances with proven efficacy have been bound by a linker to a ligand showing high affinity for membrane receptors overexpressed in tumor cells. In our case, methotrexate was selected as the active substance.

Methotrexate (MTX) is a well-known and widely characterized anti-neoplastic agent chemically suitable for conjugation with peptides. This compound is a strong inhibitor of the enzyme dihydrofolate reductase, which is responsible for the recycling of 7,8-dihydrofolate to its reduced, physiologically active form of 6-(R)-tetrahydrofolate. Although MTX does not affect all types of cancer, this cytotoxic agent has been used successfully in chemotherapy for over 4 decades. While attempting to identify more potent MTX analogues, it has been found that changes can be made to the side chain of its glutamic acid moiety without loss of activity. The maintenance of activity at the t-carboxylic position of its glutamic acid residue has also been noted. This makes MTX suitable for conjugation even with large "carriers" such as carbohydrates. These conjugates can retain MTX-like antifolate activity and selectively target tumor cells with overexpressed receptors.

Methotrexate (MTX) is an antimetabolite of folic acid that binds to the folate receptor on the surface of cancer cells. It is widely used to treat a variety of malignant tumors. It is also a widely used as an anti-rheumatic drug. MTX has also a significant number of side effects that can increase liver and kidney toxicity, as it acts on the cytoplasm of cancer cells rather than the nucleus and inhibits cancer cell DNA synthesis by inhibiting dihydrofolate reductase.

MTX is currently formulated as injectable solutions and tablets for oral administration. While it is by far one of the most effective drugs in treating various tumors and rheumatoid arthritis, there are some limitations regarding the use of MTX. The pharmacokinetics of MTX are unsatisfactory and usually lead to insufficient clinical response. The bioavailability of MTX in low doses is almost complete, while in high doses it only reaches 10-20%. Large amounts of MTX administered are excreted by the kidneys in a short time, resulting in a short plasma half-life of 5-8 hours and low drug concentrations in target tissues, e.g. in tumors, inflamed joints, etc. In general, the main reason for MTX discontinuation is not its ineffectiveness, but its toxicity. Symptoms of gastrointestinal toxicity such as stomatitis, nausea, and abdominal discomfort are among the most common. While the exact mechanisms of the toxicity are still unclear, some of the side effects have been directly linked with the involvement of MTX in metabolic pathways. 7-OH-MTX, the major metabolite of MTX, is less soluble in water and may contribute to nephrotoxicity at high doses. These side effects limit the maximum dose that can be administered. Due to the increasing incidence of neoplasms, effective therapies are under constant research. One of them includes the modification of classic drugs, which allows the reduction of side effects and, at the same time, increase their therapeutic effectiveness.

To improve drug efficacy, it is necessary to develop targeted therapies that will be characterized by increased uptake of the active ingredient by cancer cells compared to normal cells [Srinivasarao M, Low PS. Ligand-Targeted Drug Delivery. Chemical Reviews. 2017; 117 (19): 12133-64]. Extensive research has been conducted in recent years to overcome these limitations using new drug delivery systems. These systems offer many benefits such as improved drug safety and efficacy, improved bioavailability, prolonged drug action in the target tissue, and improved stability of therapeutic agents against chemical and enzymatic degradation.

The metabolic properties of malignant cells differ significantly from those of normal cells, providing the potential for targeted cellular metabolism to improve the selectivity of anti-cancer drugs. One of the strategies, targeting metabolic differences of cancer cells, is glucose-drug conjugation. The increased demand for glucose by solid tumors compared to healthy tissue, first observed by Otto Warburg in the first half of the 20th century, is utilized. Elevated levels of glucose transporters (GLUTs) are a key characteristic of many cancer cells. Malignant cells have up to 12 times higher GLUT expression compared to normal cells. Glucose-conjugates are designed to be recognized by specific GLUT receptors and absorbed by cancer cells at a faster rate than by healthy cells [Calvaresi EC, Hergenrother PJ. Glucose conjugation for the specific targeting and treatment of cancer. Chem Sci. 2013; 4: 2319-33].

MTX glycoconjugates were developed to improve the specificity of the drug, overcome drug resistance and prolong its retention in the bloodstream. Selective targeting requires not only the carrier dependent uptake pathway, but also blocking other pathways such as passive diffusion.

In the case of methotrexate, the strategy for prodrug synthesis focused on developing methods of delivering a therapeutic agent using a polymer-drug conjugate. Such prepared macromolecular drugs have the advantage of increased potency, while reducing the resistance to therapy and the toxic effects of the currently used anticancer drugs [L. Abdulrahman, O. Bakare, M. Abdulrahman, The Chemical Approach of Methotrexate Targeting Frontiers in Biomedical Sciences Vol. 1, No. 2, 2016, pp. 50-73].

The application P. 426731 presents a methotrexate-glucose glycoconjugate derivative in which D-glucose is linked to the drug amino groups via a linker. However, the derivative proposed there and the method of its preparation have some limitations. The yield of the expected product did not exceed several percent. Moreover, obtaining a high-purity preparation requires multiple purification by chromatography. The obtained compound is very sensitive to radiation and temperature, which significantly affects its stability.

The object of the invention is a compound of Formula **1** for use in chemotherapy.

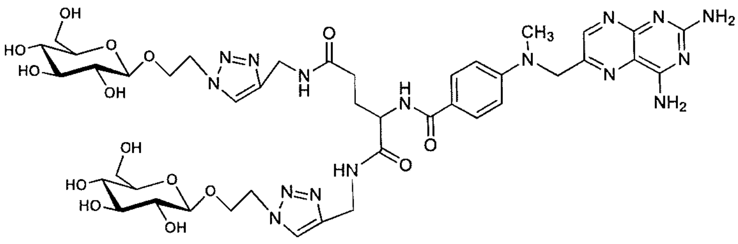

**Formula 1**

A further object of the invention is a compound of Formula **1** for use in the treatment and prevention of neoplasms.

Preferably, the compound for use according to the invention is obtained for use in the treatment and prevention of neoplasms selected from: skin cancer, colorectal cancer, breast cancer, gastric cancer, sarcoma and lung cancer.

Another object of the invention is a method for the preparation of the compound of Formula **1,** characterized in that it comprises the following steps:

a) reaction of methotrexate with *N*-propargylamine is carried out, preferably in the presence of *N,N*-diisopropylcarbodiimide (DIC) and 1-hydroxybenzotriazole (HOBT), and then diamide compound of Formula **2** is isolated from the reaction mixture:

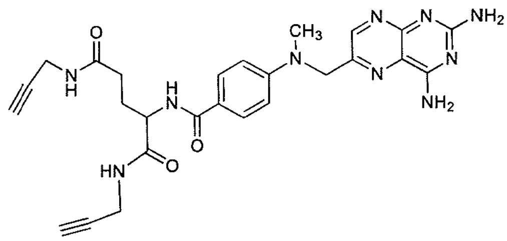

**Formula 2**

b) reaction of propargylamide derivative of methotrexate of Formula **2** with a sugar azide of Formula **3** is carried out:

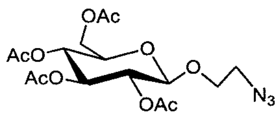

**Formula 3**

preferably in the presence of sodium ascorbate and copper sulfate, and then the compound of Formula **4** is isolated from the reaction mixture:

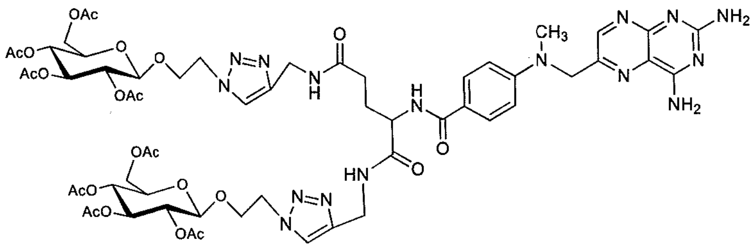

**Formula 4**

c) the deprotection reaction of the glycoconjugate of Formula **4** is carried out in a solution of sodium methoxide in methanol and the compound of Formula **1** is isolated.

In the method according to the invention, in step a), the reaction is preferably carried out in a mixture containing methotrexate (MTX), *N,N*-diisopropylcarbodiimide (DIC), 1-hydroxybenzotriazole (HOBT) and *N*-propargylamine in a mole ratio of MTX/DIC/HOBT/propargylamine of 1/1.1/0.5/2.2.

In the method according to the invention, in step a) the methotrexate is preferably dissolved in a mixture of DMF and DCM.

In the method according to the invention, in step a), the compound of Formula **2** is isolated by column chromatography, preferably on silica gel, using the $CHCl_3$:$CH_3OH$ system in a volume ratio of 50:1 to 10:1 as eluent.

In the method of the invention, in step b), the compound of Formula **4** is isolated by column chromatography, preferably on silica gel, using the $CHCl_3$:$CH_3OH$ system in a volume ratio of 20:1 to 5:1 as eluent.

In the method of the invention, in step b), the compound of Formula **2** is dissolved in an isopropyl alcohol/THF mixture in a 1/1 volume ratio.

In the method of the invention, in step c), the glycoconjugate of Formula **4** is dissolved in methyl alcohol.

In the method of the invention, in step c), neutralization of the reaction mixture is carried out using a cation exchanger in the hydrogen form.

In the method of the invention, in step c), the reaction is carried out by protecting the reaction vessels from light.

Another object of the invention is the compound of Formula **2.**

Further object of the invention is the compound of Formula **4.**

The prodrug according to the invention comprises D-glucose linked via a difunctional linker to methotrexate. The combination of the difunctional linker with sugar and methotrexate was designed to release MTX upon introduction of the prodrug into the tumor cell by hydrolysis of the glycosidic and amide linkages catalyzed by glycosylhydrolase and peptidase hydrolytic enzymes. The sugar substrate is 3-azidopropyl tetra-*O*-acetyl-β-D-glucoside. An intermediate substrate, a methotrexate derivative, is a diamide, the product of reaction of *N*-propargylamine with the carboxyl groups of methotrexate. When planning the glycoconjugate synthesis, a number of assumptions were made that determine the effectiveness of the method. Thus, due to the observed isomerization of the glutamic acid residue in carboxyl functionalization reactions, this step of amide bond formation was planned to be a carbodiimide catalyzed condensation reaction in the presence of 1-hydroxybenztriazole. Linkage of the substrates was planned to be a 1,3-dipolar cycloaddition of the azide to a terminal triple bond to form a triazine. The final stage of the synthesis is the deacylation of the sugar unit. The conventional method is an alcoholysis reaction catalyzed by sodium methoxide under the conditions described by Zemplen. Under the selected conditions, sugar deprotection did not lead to the lysis of the amide bond. The product was isolated by column chromatography.

The subject of the invention is shown in an exemplary embodiment and in the attached figures, where:

figure 1 shows the effect of Glu-MTX and methotrexate on the viability of SCC-25 cells (skin cancer),
figure 2 shows the effect of Glu-MTX and methotrexate on the viability of A427 cells (lung cancer),
figure 3 shows the effect of Glu-MTX and methotrexate on the viability of MCF-7 cells (breast cancer),
figure 4 shows the effect of Glu-MTX and methotrexate on the viability of MG-63 cells (osteosarcoma),
figure 5 shows the effect of Glu-MTX and methotrexate on the viability of DLD-1 cells (colorectal cancer),
figure 6 shows the effect of Glu-MTX and methotrexate on the viability of healthy WI38 human cells (fibroblasts),
figure 7 shows the results of MS analysis for MCF-7 breast cancer tumor cells,
figure 8 shows the results of MS analysis for A427 lung cancer tumor cells,

The prodrug synthesis is detailed in the scheme below.

**Example 1**

**Chemical synthesis**

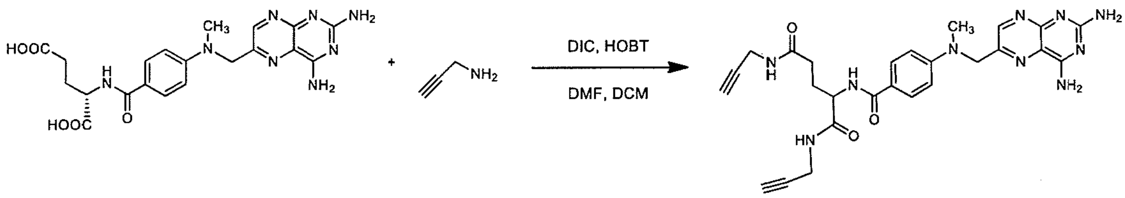


The method of obtaining methotrexate prodrug consists of the following steps:

1) amide synthesis - dissolving MTX in dry DMF, heating at a temperature of 20°C to 30 °C, to dissolve MTX, adding methylene chloride to obtain a saturated MTX solution, then cooling to 0 °C, adding *N,N*-diisopropylcarbodiimide (DIC), 1-hydroxybenzotriazole (HOBT) and propargylamine. The mole ratio of MTX/DIC/HOBT/amine is 1/1.2/0.5/2.2. The amidation reaction was carried out at room temperature while stirring the contents of the vessel for 10 days. After concentration of the reaction mixture, the product was then isolated by column chromatography on silica gel.

2) glycoconjugate synthesis by 1,3-dipolar cycloaddition: the separated, chromatographically pure diamide was dissolved in isopropyl alcohol/THF (1/1; v/v). 3-Azidopropyl tetra-O-acetyl-β-D-glucopyranoside was added to the resulting solution and stirred until a yellow solution was obtained. Thereafter, an aqueous solution of sodium ascorbate and an aqueous solution of copper (II) sulfate were added. The reaction was carried out at room temperature for 24 hours. After completion of the reaction (complete conversion of the diamide), the precipitate was filtered off, the filtrate was concentrated, and the product was isolated by column chromatography on silica gel.

3) methanolysis - deacylation, the obtained glycoconjugate was dissolved in methyl alcohol (5 ml), a solution of sodium methoxide in methanol (1 ml) was added, stirred at room temperature for 30 minutes, hydrogen cation exchanger was added to neutralize the solution, the cation exchanger was filtered off, washed with methanol, the filtrates were concentrated under reduced pressure to afford chromatographically pure methotrexate conjugate prodrug as yellow solid.

The described reactions were carried out by protecting the reaction vessels from light.

**Step I: Synthesis of the propargylamide derivative of methotrexate**

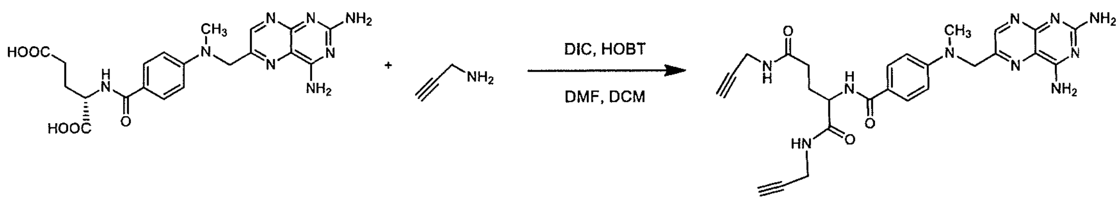


Methotrexate (500 mg, 1.10 mmol) was dissolved in anhydrous DMF (10 mL) and anhydrous DCM (2 mL). The flask was protected from light and placed in an ice/water bath. DIC (210 µL, 1.34 mmol) was added dropwise to the reaction mixture at 0 °C, HOBT (77 mg, 0.57 mmol) was added followed by propargylamine (155 µL, 2.42 mmol). The reaction was carried out at room temperature for 10 days. After quenching the reaction, the contents of the flask were concentrated under reduced pressure. The crude product was purified by column chromatography on silica gel (50:1 to 10:1 $CHCl_3$:$CH_3OH$ gradient elution) to give a yellow solid (407 mg, 70%; m.p.: 105 °C, $\alpha_D^{23} = 16$ (c = 1.0, DMSO)).

$^1H$ NMR (400 MHz, DMSO-d6): δ 1.80-2.05 (m, 2H, 2x$CH_{MTX}$), 2.07-2.24 (m, 2H, $CH_{2MTX}$), 3.06 (m, 1H, CH), 3.16 (m, 1H, CH), 3.22 (s, 3H, $CH_{3MTX}$), 3.80-3.85 (m, 4H, 2x$CH_2$), 4.33 (m, 1H, $CH_{MTX}$), 4.80 (s, 2H, $CH_{2MTX}$), 6.78-6.85 (m, 4H, 2xH-$PH_{MTX}$, $NH_{2MTX}$), 7.63 (bs, 1H, $NH_{MTX}$), 7.72-7.77 (m, 2H, 2xH-$PH_{MTX}$), 7.85 (bs, 1H, $NH_{MTX}$), 8.09 (m, 1H, $NH_{MTX}$), 8.23-8.31 (m, 2H, 2xNH), 8.58 (s, 1H, H-$7_{MTX}$).

$^{13}C$ NMR (100 MHz, DMSO-d6): δ 27.36, 27.76, 27.94, 31.77, 38.87, 52.90, 54.82, 72.81, 72.83, 81.13, 81.17, 110.92, 110.99, 121.17, 121.46, 128.90, 128.97, 146.61, 149.06, 150.82, 152.54, 160.82, 162.64, 166.07, 171.38, 171.58.

HRMS (ESI-TOF): calculated for $C_{26}H_{28}N_{10}O_3$ ($[M + H]^+$): *m/z* 529.2424, found: *m/z* 529.2421.

**Step II: Synthesis of glycoconjugate derivative of methotrexate**

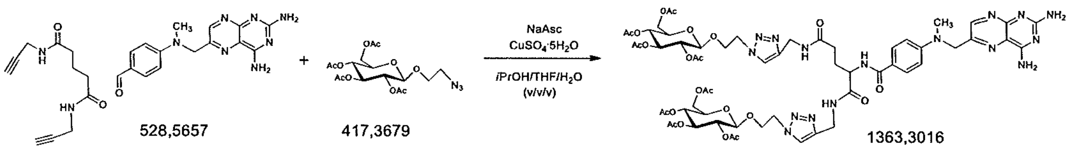


The propargylamide derivative of methotrexate (407 mg, 0.77 mmol) obtained in the previous step and sugar azide (643 mg, 1.54 mmol) were dissolved in a mixture of *i*-PrOH (6 mL) and THF (6 mL). Catalytic systems were prepared in two vials: sodium ascorbate (123 mg, 0.62 mmol) dissolved in $H_2O$ (3 mL) and $CuSO_4 \cdot 5H_2O$ (77 mg, 0.31 mmol) dissolved in $H_2O$ (3 mL), they were mixed together and added to the reaction mixture. The reaction was carried out at room temperature for 24 h. After completion of the reaction, the contents of the flask were concentrated under reduced pressure. The crude product was purified by silica gel column chromatography (20:1 to 5:1 $CHCl_3$:$CH_3OH$ gradient elution) to give a yellow solid (724 mg, 69%; m.p.: 114 °C, $\alpha_D^{23} = -6$ (c = 1.0, DMSO).

$^1H$ NMR (400 MHz, DMSO-d6): δ 1.87-1.91 (m, 2H, 2x$CH_{MTX}$), 1.92, 1.98, 2.01, 2.02 (4s, 24H, 8x$CH_3CO$), 2.11-2.24 (m, 2H, $CH_{2MTX}$), 3.21 (s, 3H, **$CH_{3MTX}$),** 3.84-3.93 (m, 2H, $CH_2NH$), 3.97 (ddd, 2H, *J* = 2.4 Hz, *J* = 4.9 Hz, *J* = 9.8 Hz, 2xH-$5_{glu}$), 4.03 (dd, 2H, *J* = 2.4 Hz, *J* = 12.3 Hz, 2xH-$6a_{glu}$), 4.05-4.12 (m, 2H, $CH_2NH$), 4.17 (dd, 2H, *J* = 4.9 Hz, *J* = 12.3 Hz, 2xH-$6b_{glu}$), 4.22-4.32 (m, 4H, 2x$CH_2O$), 4.36 (m, 1H, $CH_{MTX}$), 4.42-4.54 (m, 4H, 2x$CH_2N$), 4.72 (dd-t, 2H, *J* = 8.2 Hz, *J* = 9.4 Hz 2xH-$2_{glu}$), 4.79 (s, 2H, $CH_{2MTX}$), 4.82 (d, 2H, *J* = 8.2 Hz, 2xH-$1_{glu}$), 4.89 (dd-t, 2H, *J* = 9.4 Hz, *J* = 9.4 Hz, 2xH-$3_{glu}$), 5.22 (dd-t, 2H, *J* = 9.4 Hz, *J* = 9.8 Hz, 2xH-$4_{glu}$), 6.65 (bs, 2H, $NH_{2MTX}$), 6.81 (m, 2H, 2xH-$PH_{MTX}$), 7.74 (m, 2H, $NH_{2MTX}$), 7.78 (m, 2H, 2xH-$PH_{MTX}$), 8.08 (m, 1H, $NH_{MTX}$), 8.26-8.38 (m, 2H, 2xNH), 8.56 (s, 2H, 2xH-5triaz), 8.59 (s, 1H, H-$7_{MTX}$).

$^{13}C$ NMR (100 MHz, DMSO-d6): δ 20.19, 20.22, 20.34, 20.47, 27.55, 31.91, 34.15, 38.89, 49.11, 53.08, 54.83, 61.62, 67.38, 68.08, 70.52, 70.64, 71.91, 99.13, 110.99, 121.21, 121.41, 122.98, 128.96, 144.67, 146.16, 149.10, 150.86, 154.71, 162.55, 162.66, 166.09, 168.95, 169.23, 169.48, 170.02, 171.62, 171.76.

HRMS (ESI-TOF): calculated for $C_{58}H_{74}N_{16}O_{23}$ ($[M + H]^+$): *m/z* 1363.5191, found: *m/z* 1363.5137.

**Step III: Methanolysis, removal of acetyl groups**

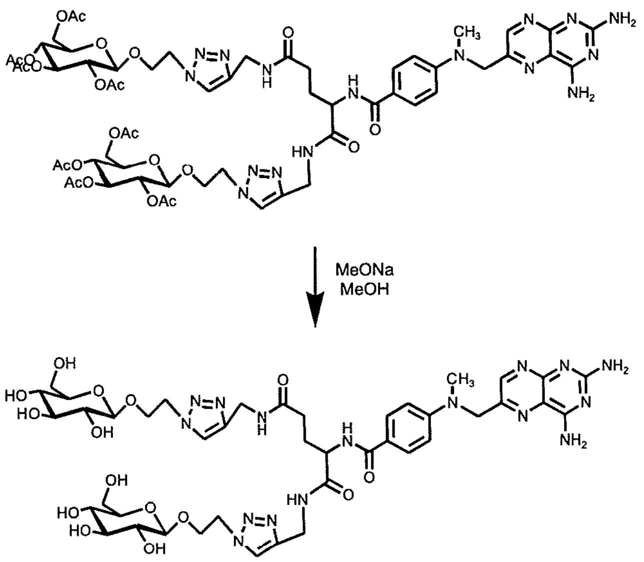


A weighed aliquot of the obtained glycoconjugate (300 mg, 0.22 mmol) was dissolved in methanol (20 mL), and then a 1M solution of sodium methoxide in methanol (600 μL, 0.6 mmol) was added. The reaction was carried out at room temperature for 3 h. After completion of the reaction, the contents of the flask were neutralized with Amberlyst-15 ion exchange resin, which was then filtered, and the filtrate was concentrated under reduced pressure to give the deprotected glycoconjugate as a yellow solid (183 mg, 81%; m.p.: 107-108 °C, $\alpha_D^{23} = 42$ (c = 1.0, DMSO).

$^{1}H$ NMR (400 MHz, DMSO-d6): δ 1.85-2.10 (m, 2H, 2x$CH_{MTX}$), 2.13-2.28 (m, 2H, $CH_{2MTX}$), 3.21 (s, 3H, $CH_{3MTX}$), 2.93-3.01 (m, 2H, 2xH-$2_{Glu}$), 3.03-3.08 (m, 2H, 2xH-$4_{Glu}$), 3.09-3.16 (m, 4H, 2xH-$3_{Glu}$, 2xH-$5_{Glu}$), 3.39-3.49 (m, 2H, 2xH-$6a_{Glu}$), 3.63-3.70 (m, 2H, 2xH-$6b_{Glu}$), 3.83-3.93 (m, 4H, $CH_2NH$), 4.01-4.10 (m, 4H, 2x$CH_2$), 4.19-4.24 (m, 2H, $CH_2$), 4.25-4.32 (m, 4H, 2xH-$1_{Glu}$, 2x$CH_2$), 4.38 (m, 1H, $CH_{MTX}$), 4.47-4.56 (m, 4H, 4xOH), 4.78 (s, 2H, $CH_{2MTX}$), 6.60 (bs, 2H, $NH_{2MTX}$), 6.81 (m, 2H, 2xH-$PH_{MTX}$), 7.66 (bs, 2H, $NH_{2MTX}$), 7.74 (m, 2H, 2xH-$PH_{MTX}$), 8.15 (m, 1H, $NH_{MTX}$), 8.32-8.44 (m, 2H, 2xNH), 8.47 (s, 2H, 2xH-$5_{triaz}$), 8.56 (s, 1H, H-$7_{MTX}$).

$^{13}C$ NMR (100 MHz, DMSO-d6): δ 30.24, 31.99, 34.21, 34.31, 38.62, 48.31, 49.31, 51.01, 54.55, 60.77, 66.99, 69.71, 73.00, 76.35, 76.73, 102.66, 110.74, 111.70, 120.34, 121.54, 123.25, 128.76, 144.35, 145.70, 148.86, 150.88, 154.88, 162.39, 162.55, 165.58, 171.57, 172.61.

HRMS (ESI-TOF): calculated for $C_{42}H_{58}N_{16}O_{15}$ ($[M + H]^+$): *m/z* 1027.4346, found: *m/z* 1027.5416.

**Example 2**

**Analysis of the *in vitro* biological activity of GLU-MTX compared to free MTX on solid tumor cell lines**

**Analysis of the cytotoxicity of the compound according to the invention (MTT test)**

Cells were seeded in a 96-well plate at 5-10 x $10^3$ cells per well. After 24 hours, the culture medium was decanted and 100 μL of fresh medium was added to the control wells, while the medium with test compounds dissolved in DMSO was added to the test wells. The plates were wrapped in foil and placed in an incubator. After 72 hours of incubation, the medium was decanted and 100 μL of a solution of yellow soluble 3-(4,5-dimethyl-2-thiazolyl)-2,5-diphenyl-2H-tetrazolium bromide (MTT) at a concentration of 0.5-1 mg/mL was added. After 4 hours of incubation, the MTT solution was decanted and 50 μL/well of DMSO was added. After 3 hours, the optical density of the dissolved formazan was analyzed colorimetrically in a BioTek plate reader. The concentration of DMSO in the tested samples did not exceed 0.2%.
Since living cells reduce MTT to formazan, the optical density (OD) is highest in the control (C) wells which accounted for 100% of cell viability. Viablity in wells incubated with compound (COMP) was calculated using the formula:

$$X = 100\% \text{ x OD COMP/OD C}$$

The results reported are the mean value of triplicate cytotoxicity analysis.
Cell viability studies using the MTT assay on cell lines of numerous cancers, including breast cancer, colorectal cancer, lung cancer, skin cancer, and bone sarcoma have shown that Glu-MTX is cytotoxic *in vitro.* **Figures 1-6** and Table 1 show the comparative results of a cell viability assessment (IC50) study for various tumor cell lines and normal fibroblasts treated with methotrexate alone or a glucose-methotrexate derivative according to the invention (compound of Formula 1). The cytotoxic effect of the glucose-methotrexate conjugate is similar to that of unmodified methotrexate. The study used: GLU-MTX 10 μM, Methotrexate 10 μM; incubation 72 hours; MTT analysis.

**Table 1**

| Compound | IC50 values (μM) for individual cell lines | | | | | | |
|---|---|---|---|---|---|---|---|
| | MCF-7 (breast) | DLD-1 (large intestine) | SCC25 (skin) | A427 (lung) | MG-63 (bone sarcoma) | SW480 (large intestine) | WI38 (healthy fibroblasts) |
| GLU-MTX | 16.24±0.2 2 | 9.81±0.32 | 71.42±2.1 2 | 7.35±0.0 6 | 79.15±0.44 | 6.8±0.36 | 52.15±1.22 |
| MTX | 9.61±0.12 | 6.85±0.21 | 80.64±1.8 7 | 6.02±0.3 2 | 60.97±0.76 | 4.82±0.3 1 | 16.88±1.94 |

**MS analysis**

Cells were seeded in a 6-well plate at 2.5 x $10^5$ cells/well and 1 mL/well. When the cells reached 80% confluence, the culture medium was decanted and 1 mL per well of fresh medium to the control wells or 1 mL per well of the test medium with methotrexate at a concentration of 10 μM or a derivative of methotrexate at a concentration of 10 μM, respectively, was added. The plate was wrapped in foil and incubated for 2 hours in an incubator. After this time, the medium was decanted, and each well was washed with 2x1 mL of PBS at room temperature. Then, the plate was placed on ice and 500 μL of a 3:1 mixture of methanol and ultrapure water at -20 °C were applied to each well. The mixture was pipetted several

times in each well and collected in a test tube. Then, the procedure was repeated and the prepared samples (each with a volume of 1 mL) were placed in a lowtemperature freezer at -80 °C. The next day, the samples were concentrated in a vacuum concentrator until the samples were completely dry. Prior to analysis, the solvent for MS analysis (Xevo G2 Q-TOF MS) was added to the sample. In figure 7 it can be seen that in case of MTX the surface area of MCF7 tumor cells grown with MTX is about 308 units, and when using a compound according to the invention (identified as 77_1 in Figure 7) it is 525, i.e. about 1.7 times less than in the case of a conjugate. In figure 8, by analogy for A427 cells, the surface area in case of MTX is 1.7 times smaller compared to that of the conjugate. Neoplastic cells, due to the overexpression of the GLUT-1 transporter, more efficiently accumulate intracellularly methotrexate-glucose conjugate (GLU-MTX) after 2 hours, compared to free methotrexate.

**Example 3**

***In vivo* evaluation of the pharmacokinetic profile of the conjugate according to the invention**

The pharmacokinetic profile of the glucose-methotrexate derivative according to the invention (GMTX; chemical formula: $C_{42}H_{58}N_{16}O_{15}$) was examined in relation to the profile of the classical form of this drug (MTX) after a single intravenous administration into the tail vein. The solutions administered to the animals were prepared on the day of administration.

The GMTX was an orange powder and was odorless. The compound was stored at 4-8 °C in the closed original package.

GMTX was administered to mice intravenously via the tail vein, in a single dose, in the form of solutions (with concentrations ensuring that the animals are dosed appropriately) prepared in 5% DMSO and saline in the 1:20 ratio, in a volume not exceeding 0.2 $cm^3$/mouse. The vehicle of the test material was a sodium chloride solution for injection with DMSO (the ratio of DMSO and saline was 1:20 - 5% DMSO).

The methotrexate (chemical formula: $C_{20}H_{22}N_8O_5$; CAS No: 59-05-2) used in the study was from EBEWE Pharma Ges.m.b.H.Nfg. (Methotrexat-Ebewe, 100 mg/$cm^3$) and was in the form of concentrate for solution for infusion.

The study was carried out on 40 sexually mature male house mice (Mus musculus; BALB/ccmdb inbred strain) weighing 18.05 - 20.80 g (differing by no more than ± 20%). The experimental animals came from the breeding farm of the Medical University in Bialystok, from the Experimental Medicine Center.

The selection of animals for the study was made by random selection in the breeding barrier. Before transferring the mice from the breeding barrier to the experimental barrier, the veterinarian performed a health evaluation of the animals in the breeding barrier to qualify them for the experiment. The mice were acclimated for 5 days before the test material was administered. During the experiment, the animals were kept in a specific pathogen free (SPF) status in an individually ventilated cage system (IVC; models 1284 and 1285 from Tecniplast S.p.A, Italy). The equipment and materials used in the study were washed and sterilized. The experimental animals were fed with sterile (disinfected with hydrogen peroxide) living fodder for mice (ssniff Spezialdiäten GmbH) and sterile, contaminant-free drinking water throughout the experiment. The mice had unrestricted access to feed and water.

Mice were administered intravenously (i.v.) GMTX and MTX and blood was collected (to obtain plasma) at 4 time points after injection (1 h, 2 h, 4 h and 8 h), as well as in the so-called time 0 (blood sampling from animals that received neither MTX nor GMTX). The study was carried out on 40 male BALB/ccmdb mice. Sixteen males were administered MTX at a dose of 32.5 mg/kg body weight (½ $LD_{50}$ for MTX for mice when administered intravenously, $LD_{50}$ = 65 mg/kg b.w.). Another 16 males were administered GMTX at a dose of 400 mg/kg body weight, constituting ½ the MTD of this compound (determined in our study with the code T425/001/2020), and the remaining 8 subjects were not administered either classic MTX nor its glycoconjugate (GMTX). Two hours prior to treatment with MTX or GMTX, the animals were stopped feeding, and 30 minutes after dosing, the animals were re-fed.

MTX and GMTX were administered to mice as solutions prepared for injection at concentrations of 0.783 mg/0.2 $cm^3$ and 9.04 mg/0.2 $cm^3$, respectively.

Immediately after anaesthesia (inhalation of isoflurane), at specified times, i.e. at 0, 1, 2, 4 and 8 h, the maximum volume of blood was collected from each animal into test tubes with dipotassium edetate ($K_2$-EDTA). Complete bleeding resulted in the death of the animal. Whole blood was centrifuged (3000 revolutions for 15 minutes) using a 5810R centrifuge from Eppendorf AG (Germany) to obtain plasma, which was immediately frozen in Eppendorf tubes at -80 ± 5 °C.

Plasma samples were thawed at 6 °C. To 100 μL of plasma were added 10 μL of an internal standard (IS) with a concentration of 2 μM (63/2 conjugate). Then 400 μL of methanol (-20 °C) was transferred to the test tubes and the whole was shaken for 5 minutes at 6 °C. In the next step, the tubes were centrifuged (22,000 rcf, 4 °C, 7 minutes), and the resulting supernatant was transferred to polypropylene chromatographic vessels and analyzed by LC-MS. A standard curve for the determination of MTX and GMTX in mouse plasma was prepared. The concentration of MTX and GMTX in the plasma of the mice was determined using a mass spectrometer (Xevo G2 Q-TOF) coupled to the Waters Acquity UPLC liquid

chromatograph (LC-MS). MS measurements were performed in the high sensitivity mode (Sensitivity Mode) and in positive ion mode. In order to ensure high accuracy of the m/z value measurement, LockSpray was used - a leucine enkephalin with a concentration of 50 pg/$cm^3$, which was continuously fed to the ion source. The following operating parameters for the mass spectrometer ion source were used: Voltage applied to the capillary (kV) 0.5; Voltage applied to the cone (V) 15; Ion source temperature (°C) 125; Desolvation Temperature (°C) = 450; Shielding gas flow (L/h) 65; Desolvation gas flow (L/h) 800.

The results of MTX and GMTX concentrations in the plasma of mice were statistically analyzed using the Statistica 13 computer program. The results were expressed as the mean concentration (± standard deviation - SD) at individual time points. The non-parametric Kruskal-Wallis test was used to evaluate changes in plasma MTX and GMTX concentrations up to 8 hours after intravenous administration. Differences are considered statistically significant at $p<0.05$.

The analysis of changes in MTX and GMTX concentrations showed that MTX, as well as a unique derivative of this drug and glucose - methotrexate glycoconjugate, are quickly eliminated from the systemic circulation within the first 4 hours after intravenous administration.

In addition, the elimination of GMTX from the systemic circulation two hours after intravenous administration is faster than the elimination of MTX. The elimination of GMTX compared to slow elimination of MTX after 2 hours is 6.58 times faster. The results are presented in Table 2.

**Table 2.** Mean MTX and GMTX concentration in nM at 1, 2, 4, 8 hours after intravenous administration in mouse plasma samples

| MTX 1h | MTX 2h | MTX 4h | MTX 8h | GMTX 1h | GMTX 2h | GMTX 4h | GMTX 8h |
|---|---|---|---|---|---|---|---|
| 1740.6 | 330 | 20.7 | 71.6 | 20884.7 | 311.4 | 62.5 | 12.2 |
| 2164.9 | 298.7 | 29.4 | 20.3 | 23409.9 | 1021.4 | 54.1 | 69.4 |
| 2328.5 | 222.8 | 22.6 | 7.2 | 29781.6 | 523.5 | 55.6 | 33.5 |
| 1672.4 | 311.4 | 32.1 | 23.1 | 36666.4 | | 39.9 | 6.8 |
| **1976.6** | **290.725** | **26.2** | **30.55** | **27685.65** | **618.7667** | **53.025** | **30.475** |
| 100.00% | 14.71% | 1.33% | 1.55% | 100.00% | 2.23% | 0.19% | 0.11% |

**Example 4**

**Evaluation of the acute *in vivo* toxicity of a conjugate according the invention**

The study assessed the acute toxicity of methotrexate glycoconjugate (GMTX) (single intravenous administration) with the determination of the maximum tolerated dose (MTD).

The acute toxicity assessment of GMTX was carried out using the higher - lower dose method according to OECD procedure 425, which takes into account the drive to improve animal welfare and the 3R (Replacement - Reduction - Refinement) principle.

GMTX was administered to mice intravenously via the tail vein, in a single dose, in the form of solutions (in concentrations ensuring that the animals are dosed appropriately) prepared in 5% DMSO in saline at a ratio of 1:20, in a volume not exceeding 0.2 $cm^3$/mouse. The vehicle of the test material was a sodium chloride solution for injection with DMSO (the ratio of DMSO and saline was 1:20 - 5% DMSO). The test material was stored for the duration of the test in the original packaging at a temperature of 4-8 °C. The GMTX solutions that were administered to the animals were each prepared on the day of administration. Prepared solutions of the test material and vehicle were heated to 35 °C immediately before being administered to the animals.

The study was carried out on 14 sexually mature - not younger than 8 weeks, but not older than 12 weeks, female (non-breeding and non-pregnant) domestic mice (Mus musculus; inbred BALB/ccmdb) with a body weight of 20.60 - 22.00 g (differing by no more than ± 20%). The experimental animals came from the breeding farm of the Experimental Medicine Center of the Medical University of Bialystok.

The animals were qualified for randomized testing in the breeding barrier. Before the mice were transferred from the breeding barrier to the experimental barrier, the veterinarian assessed the health of the animals to qualify them for the experiment. Mice were allowed to acclimatize for a minimum of 5 days before starting the administration of vehicle or test material. During the course of the experiment, the mortality of the mice was monitored, and general and detailed observations of the animals were made to capture any signs of toxic effects of the test material and vehicle. A total of 14 mice were used in the experiment, including 11 animals for the acute toxicity assessment of GMTX and 3 animals for the

toxicological assessment of its vehicle.

The procedure of higher - lower dose acute toxicity assessment was used, which consisted of administering the test material to a single animal at a single dose lower than the expected $LD_{50}$ value. Depending on the effects obtained after the administration of the first dose, the next subject was administered an increased or decreased dose by a constant factor. This treatment was continued until the dose was reached, where increasing resulted in death and decreased survival for 3 more animals. The mice were then observed daily for 14 days after dosing, after which the animals were subjected to a full postmortem examination with cardiac blood sampling at maximum volume and specimen collection for histopathology.

All mice administered the test material showed no signs of acute toxicity within 14 days of administration of 800 mg GMTX/kg b.w. Thus, the MTD of glucose derivative methotrexate after a single intravenous administration is greater than 800 mg/kg body weight.

Taking into account the fact that the MTD of methotrexate for mice of the BALB/c strain when administered intravenously is 94 mg/kg b.w. [Moura JA, Valduga CJ, Tavares ER, Kretzer IF, Maria DA, Maranhão RC. Novel formulation of a methotrexate derivative with a lipid nanoemulsion. Int J Nanomedicine. 2011; 6: 2285-2295] based on the results of the acute toxicity assessment for GMTX, it can be concluded that a new derivative of this drug and glucose - a glucose derivative of methotrexate, is characterized by a lower acute toxicity than the classic form of the drug, because the maximum tolerated dose of GMTX is 800 mg/kg b.w. The high tolerated dose is the greatest benefit in the case of using GMTX, as it dramatically reduces the overall toxicity of the preparation while maintaining its effect, and thus reduces the lethality caused by the toxicity.

## Claims

1. A Compound of Formula 1:

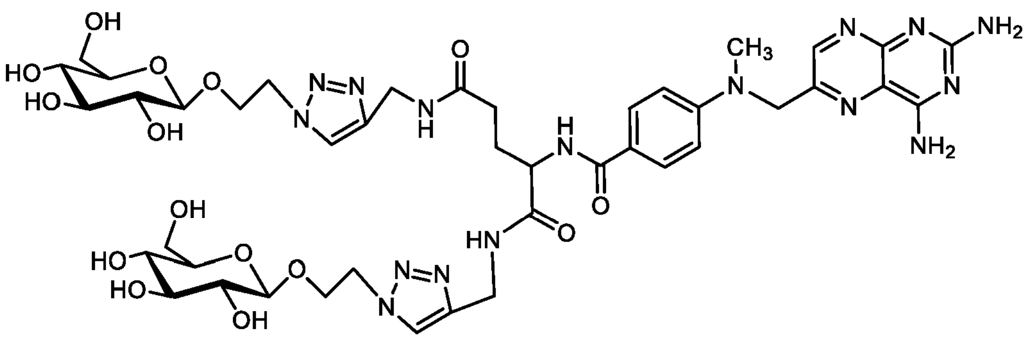

**Formula 1**

2. The compound of Formula 1 for use in medicine.

3. The compound of Formula 1 for use in the treatment and prevention of neoplasms.

4. The compound for use according to claim 3, **characterized in that** the cancer is selected from: skin cancer, colorectal cancer, breast cancer, gastric cancer, sarcoma and lung cancer.

5. A method for the preparation of a compound of Formula 1, comprising the following steps:

   a) reaction of methotrexate with *N*-propargylamine is carried out, preferably in the presence of *N,N*-diisopropylcarbodiimide (DIC) and 1-hydroxybenzotriazole (HOST), and then diamide compound of Formula 2 is isolated from the reaction mixture:

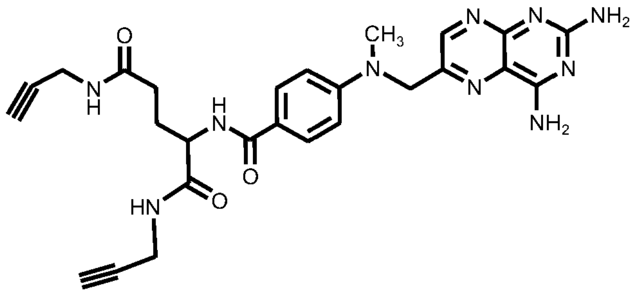

**Formula 2**

b) the azido-alkyne 1,3-dipolar cycloaddition reaction of N-propargylamide, a methotrexate derivative of Formula 2, with a sugar azide of Formula 3 is carried out:

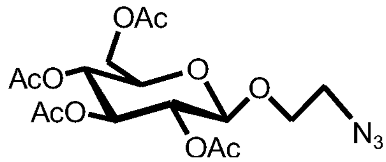

**Formula 3**

preferably in the presence of sodium ascorbate and copper sulfate, and then the compound of Formula 4 is isolated from the reaction mixture:

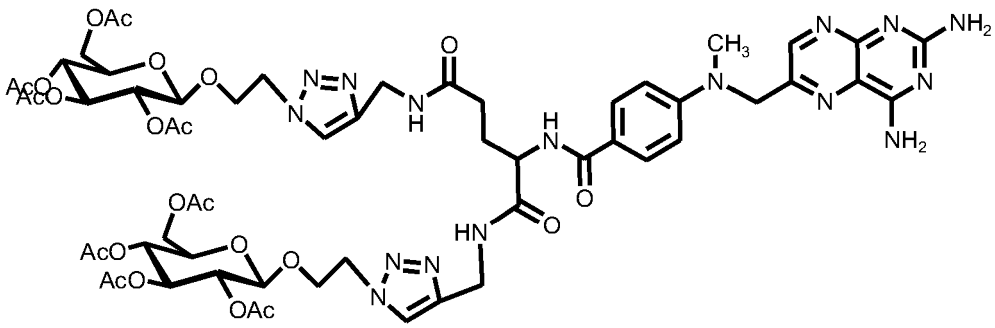

**Formula 4**

c) the deprotection reaction of the glycoconjugate of Formula 4 is carried out in a solution of sodium methoxide in methanol and the compound of Formula 1 is isolated.

**6.** The method according to claim 5, wherein in step a) the reaction is carried out in a mixture containing methotrexate (MTX), N,N-diisopropylcarbodiimide (DIC), 1-hydroxybenzotriazole (HOBT) and *N*-propargylamine in a mole ratio of MTX/DIC/HOBT/propargylamine of 1/1.1/0.5/2.2.

**7.** The method according to claim 5, wherein in step a) methotrexate is dissolved in a mixture of DMF and DCM.

**8.** The method according to claim 5, wherein in step a) the compound of Formula 2 is isolated by column chromatography, preferably on silica gel, using the $CHCl_3$:$CH_3OH$ system in a volume ratio of 50:1 to 10:1 as eluent.

**9.** The method according to claim 5, wherein in step b) the compound of Formula 4 is isolated by column chromatography, preferably on silica gel, using the $CHCl_3$:$CH_3OH$ system in a volume ratio of 20:1 to 5:1 as eluent.

**10.** The method according to claim 5, wherein in step b) the compound of Formula 2 is dissolved in an isopropyl alcohol/THF/water mixture in a 1:1:1 volume ratio.

**11.** The method according to claim 5, wherein in step c) the glycoconjugate of Formula 4 is dissolved in methyl alcohol.

**12.** The method according to claim 5, wherein in step c) neutralization of the reaction mixture is carried out using a cation exchanger in the hydrogen form.

**13.** The method according to claim 5, wherein in step c) the reaction is carried out by protecting the reaction vessels from light.

**14.** Compound of Formula 2:

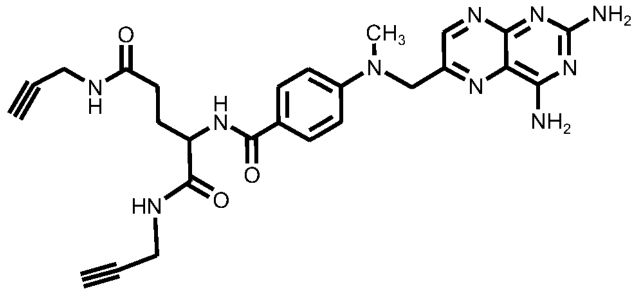

**Formula 2**

**15.** Compound of Formula 4:

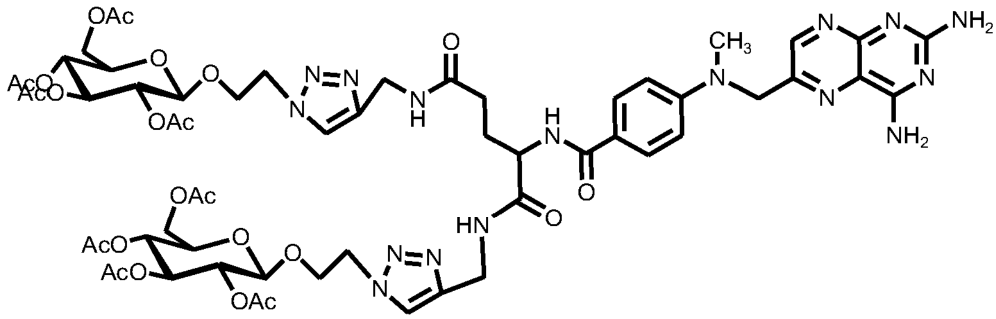

**Formula 4**

## Patentansprüche

**1.** Verbindung der Formel 1:

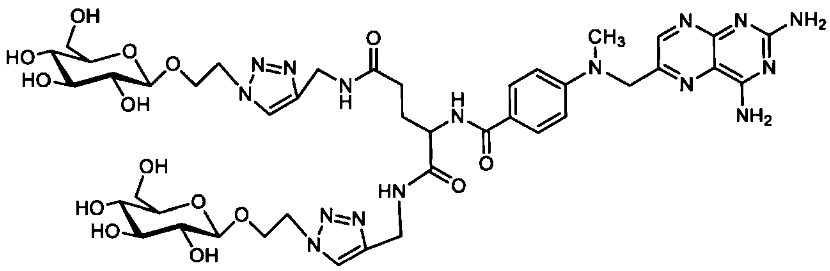

**Formel 1**

**2.** Verbindung der Formel 1, um in der Medizin verwendet zu werden.

**3.** Verbindung der Formel 1, um bei der Behandlung und Prävention von Neoplasmen verwendet zu werden.

**4.** Verbindung, zur Verwendung nach Anspruch 3, **dadurch gekennzeichnet, dass** der Krebs ausgewählt ist aus: Hautkrebs, Darmkrebs, Brustkrebs, Magenkrebs, Sarkom und Lungenkrebs.

**5.** Verfahren für die Vorbereitung einer Verbindung der Formel 1, umfassend die folgenden Schritte:

a) die Reaktion von Methotrexat mit N-Propargylamin wird vorzugsweise in Gegenwart von *N,N*-Diisopropylcarbodiimid (DIC) und 1-Hydroxybenzotriazol (HOBT) durchgeführt, und anschließend wird die Diamidverbindung der Formel 2 aus dem Reaktionsgemisch isoliert:

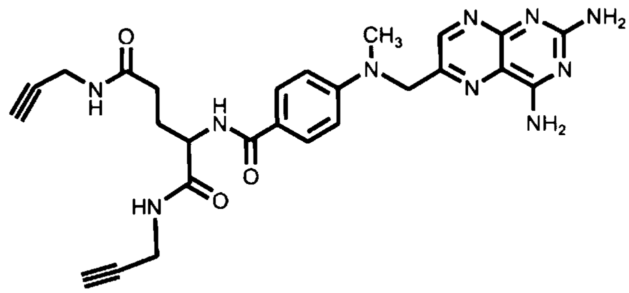

**Formel 2**

b) die Azido-Alkin-1,3-dipolare Cycloadditionsreaktion von *N*-Propargylamid, einem Methotrexatderivat der Formel 2, wird mit einem Zuckerazid der Formel 3 durchgeführt:

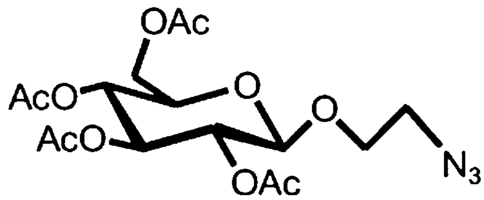

**Formel 3**

vorzugsweise in Gegenwart von Natriumascorbat und Kupfersulfat, und anschließend wird die Verbindung der Formel 4 aus dem Reaktionsgemisch isoliert:

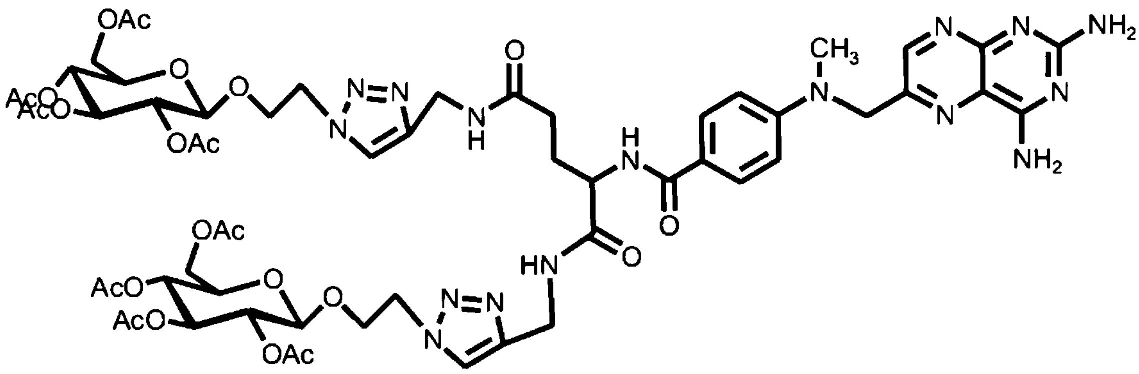

**Formel 4**

c) die Entschützungsreaktion des Glykokonjugats der Formel 4 wird in einer Lösung von Natriummethoxid in Methanol durchgeführt und die Verbindung der Formel 1 wird isoliert.

6. Verfahren nach Anspruch 5, wobei in Schritt a) die Reaktion in einer Mischung durchgeführt wird, die Methotrexat (MTX), N,N-Diisopropylcarbodiimid (DIC), 1-Hydroxybenzotriazol (HOBT) und *N*-Propargylamin in einem Molverhältnis von MTX/DIC/HOBT/Propargylamin von 1/1,1/0,5/2,2 enthält.

7. Verfahren nach Anspruch 5, wobei in Schritt a) Methotrexat in einer Mischung aus DMF und DCM gelöst wird.

8. Verfahren nach Anspruch 5, wobei in Schritt a) die Verbindung der Formel 2 durch Säulenchromatographie, vorzugsweise auf Silicagel, unter Verwendung des $CHCl_3$:$CH_3OH$-Systems in einem Volumenverhältnis von 50:1 bis 10:1 als Elutionsmittel isoliert wird.

9. Verfahren nach Anspruch 5, wobei in Schritt b) die Verbindung der Formel 4 durch Säulenchromatographie, vorzugsweise auf Silicagel, unter Verwendung des $CHCl_3$:$CH_3OH$-Systems in einem Volumenverhältnis von 20:1 bis 5:1 als Elutionsmittel isoliert wird.

10. Verfahren nach Anspruch 5, wobei in Schritt b) die Verbindung der Formel 2 in einem Gemisch aus Isopropylalkohol, THF und Wasser im Volumenverhältnis 1:1:1 gelöst wird.

11. Verfahren nach Anspruch 5, wobei in Schritt c) das Glykokonjugat der Formel 4 in Methylalkohol gelöst wird.

12. Verfahren nach Anspruch 5, wobei in Schritt c) die Neutralisation des Reaktionsgemisches unter Verwendung eines Kationenaustauschers in der Wasserstoffform durchgeführt wird.

13. Verfahren nach Anspruch 5, wobei in Schritt c) die Reaktion durchgeführt wird, indem die Reaktionsgefäße vor Licht geschützt werden.

14. Verbindung der Formel 2:

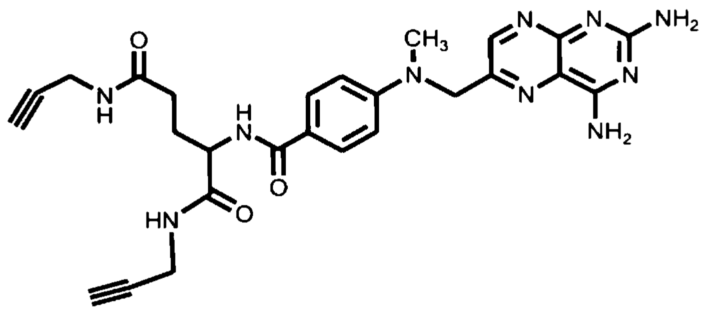

**Formel 2**

15. Verbindung der Formel 4:

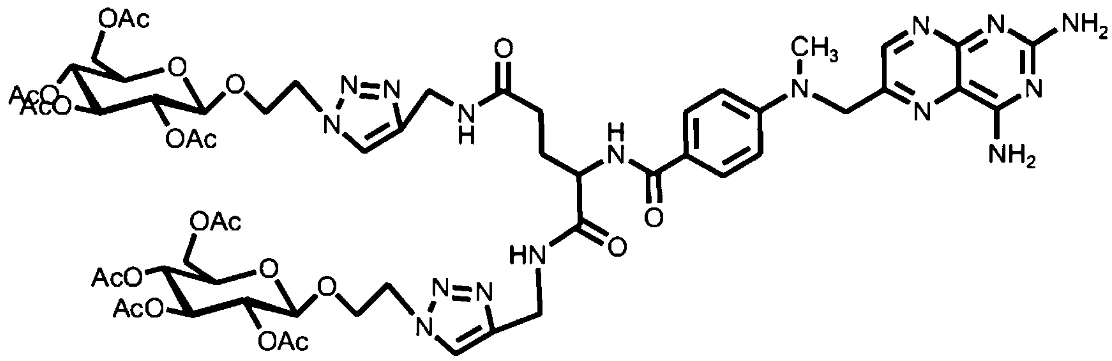

**Formel 4**

## Revendications

1. Composé de formule 1 :

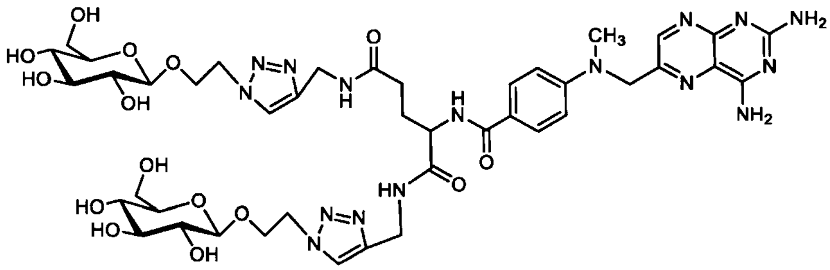

**Formule 1**

2. Composé de formule 1 pour une utilisation en médecine.

3. Composé de formule 1 pour une utilisation dans le traitement et la prévention de néoplasmes.

4. Composé pour une utilisation selon la revendication 3, **caractérisé en ce que** le cancer est sélectionné parmi : un cancer de la peau, un cancer colorectal, un cancer du sein, un cancer gastrique, un sarcome et un cancer du poumon.

5. Procédé de préparation d'un composé de formule 1, comprenant les étapes suivantes :

   a) une réaction du méthotrexate avec de la *N-propargylamine* est effectuée, de préférence en présence de *N,N-diisopropylcarbodiimide* (DIC) et de 1-hydroxybenzotriazole (HOBT), puis le composé diamide de la formule 2 est isolé du mélange réactionnel :

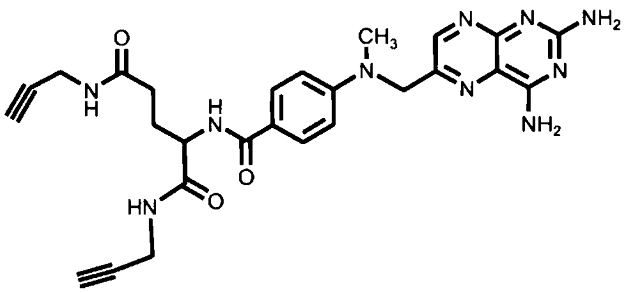

**Formule 2**

   b) la réaction de cycloaddition azido-alkyne 1,3-dipolaire de *N*-propargylamide, un dérivé de méthotrexate de formule 2, avec un azoture de sucre de formule 3 est effectuée :

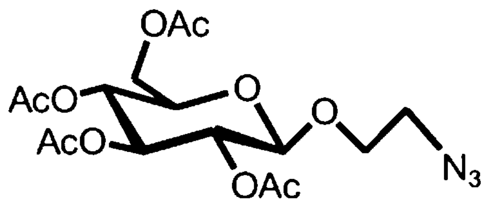

**Formule 3**

   de préférence en présence d'ascorbate de sodium et de sulfate de cuivre, puis le composé de formule 4 est isolé du mélange réactionnel :

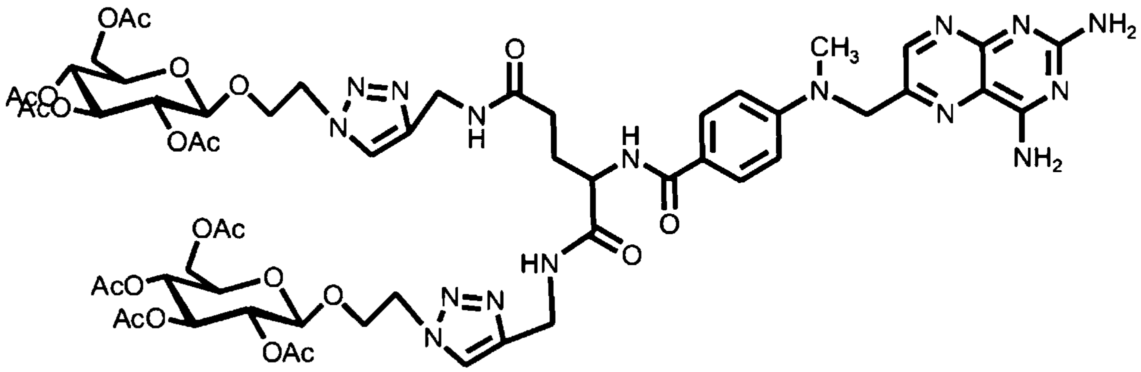

**Formule 4**

c) la réaction de déprotection du glycoconjugué de formule 4 est effectuée dans une solution de méthoxyde de sodium dans du méthanol et le composé de formule 1 est isolé.

**6.** Procédé selon la revendication 5, dans lequel, à l'étape a), la réaction est effectuée dans un mélange contenant du méthotrexate (MTX), du *N,N*-diisopropylcarbodiimide (DIC), du 1-hydroxybenzotriazole (HOBT) et de la *N*-propargylamine dans un rapport molaire de MTX/DIC/HOBT/propargylamine de 1/1,1/0,5/2,2.

**7.** Procédé selon la revendication 5, dans lequel, à l'étape a), le méthotrexate est dissous dans un mélange de DMF et de DCM.

**8.** Procédé selon la revendication 5, dans lequel à l'étape a) le composé de formule 2 est isolé par chromatographie en colonne, de préférence sur gel de silice, en utilisant le système $CHCl_3$:$CH_3OH$ dans un rapport volumique de 50:1 à 10:1 comme éluant.

**9.** Procédé selon la revendication 5, dans lequel, à l'étape b), le composé de formule 4 est isolé par chromatographie sur colonne, de préférence sur gel de silice, en utilisant le système $CHCl_3$:$CH_3OH$ dans un rapport volumique de 20:1 à 5:1 comme éluant.

**10.** Procédé selon la revendication 5, dans lequel, à l'étape b), le composé de formule 2 est dissous dans un mélange d'alcool isopropylique/THF/eau dans un rapport volumique de 1:1:1.

**11.** Procédé selon la revendication 5, dans lequel, à l'étape c), le glycoconjugué de formule 4 est dissous dans de l'alcool méthylique.

**12.** Procédé selon la revendication 5, dans lequel, à l'étape c), la neutralisation du mélange réactionnel est effectuée à l'aide d'un échangeur cationique sous forme d'hydrogène.

**13.** Procédé selon la revendication 5, dans lequel, à l'étape c), la réaction est effectuée en protégeant les récipients de réaction de la lumière.

**14.** Composé de formule 2 :

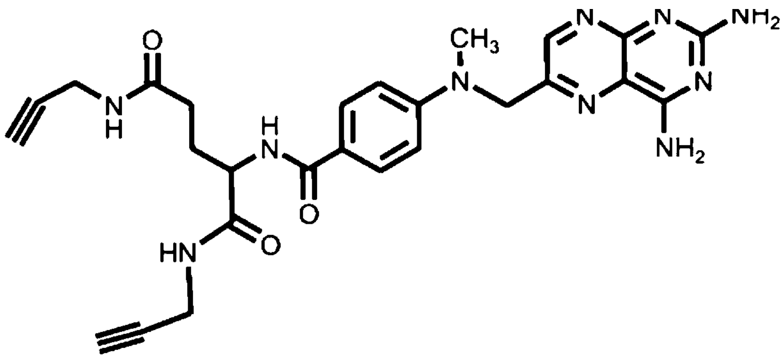

**Formule 2**

**15.** Composé de formule 4 :

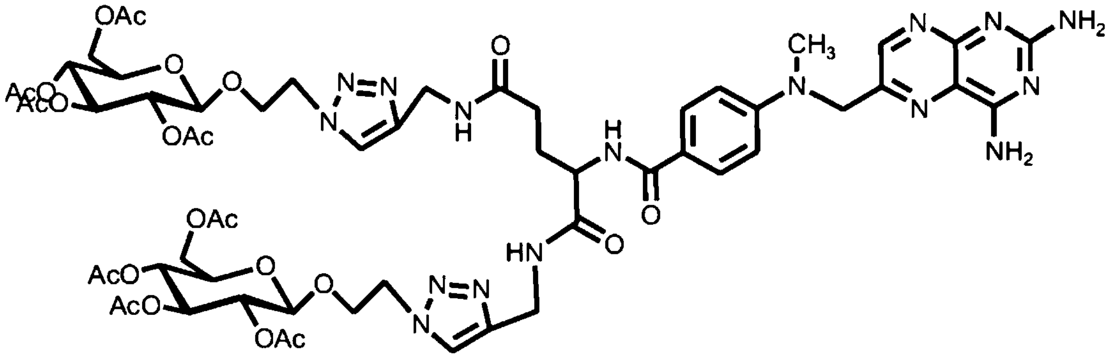

**Formule 4**

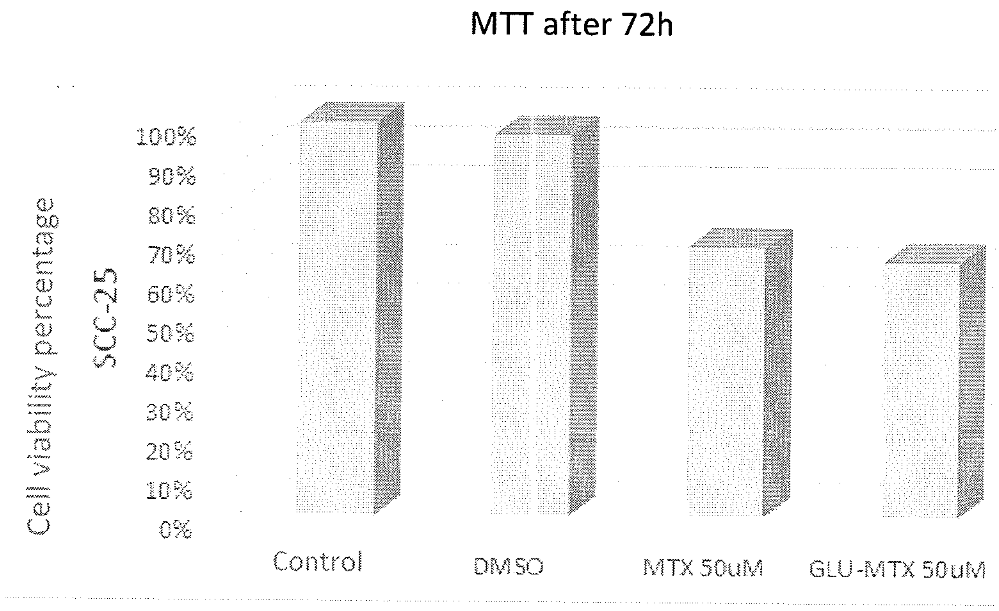


**Figure 1**

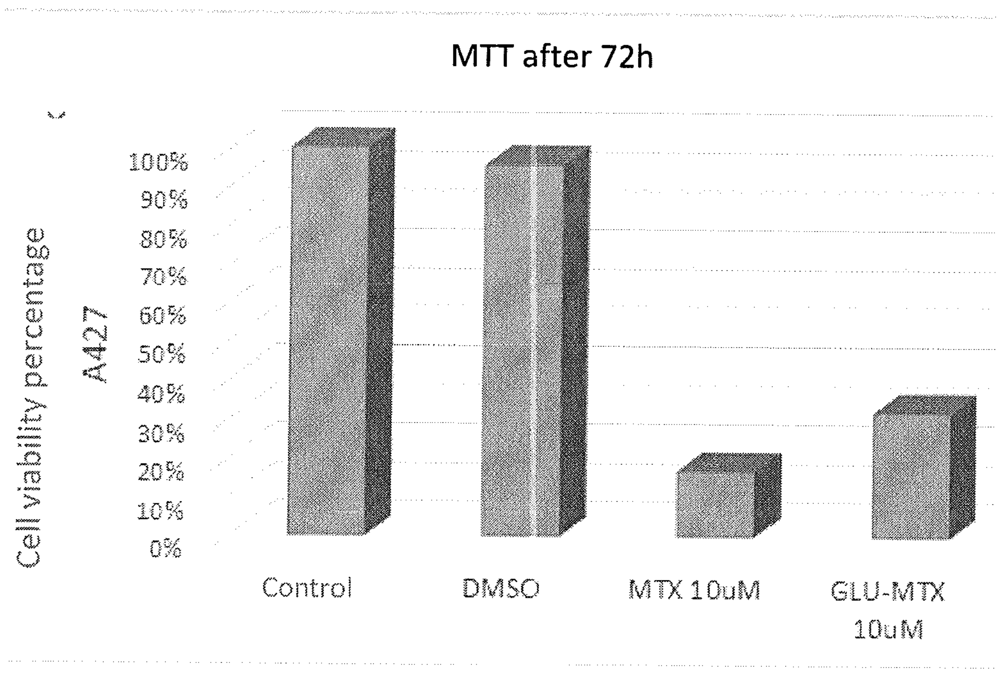


**Figure 2**

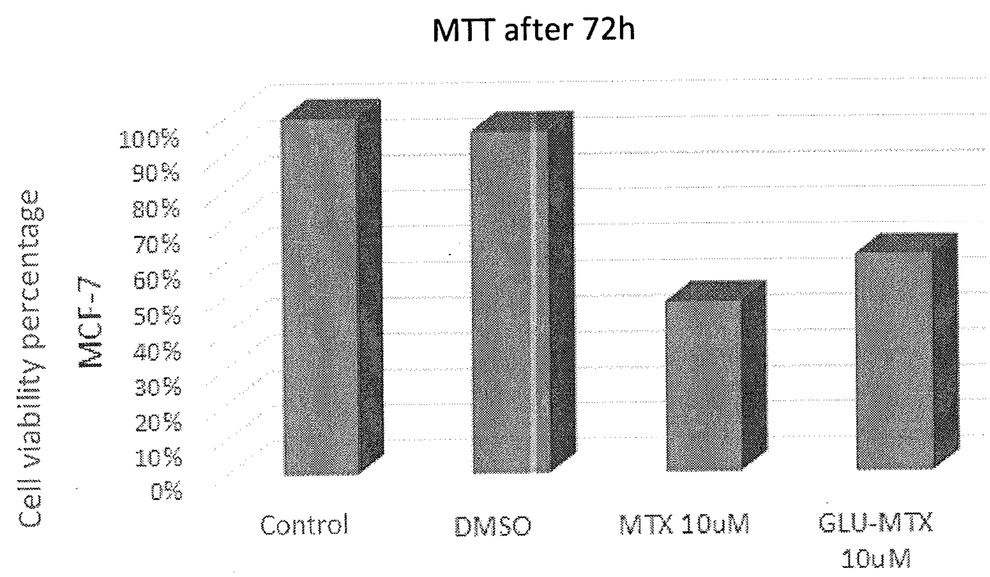


Figure 3

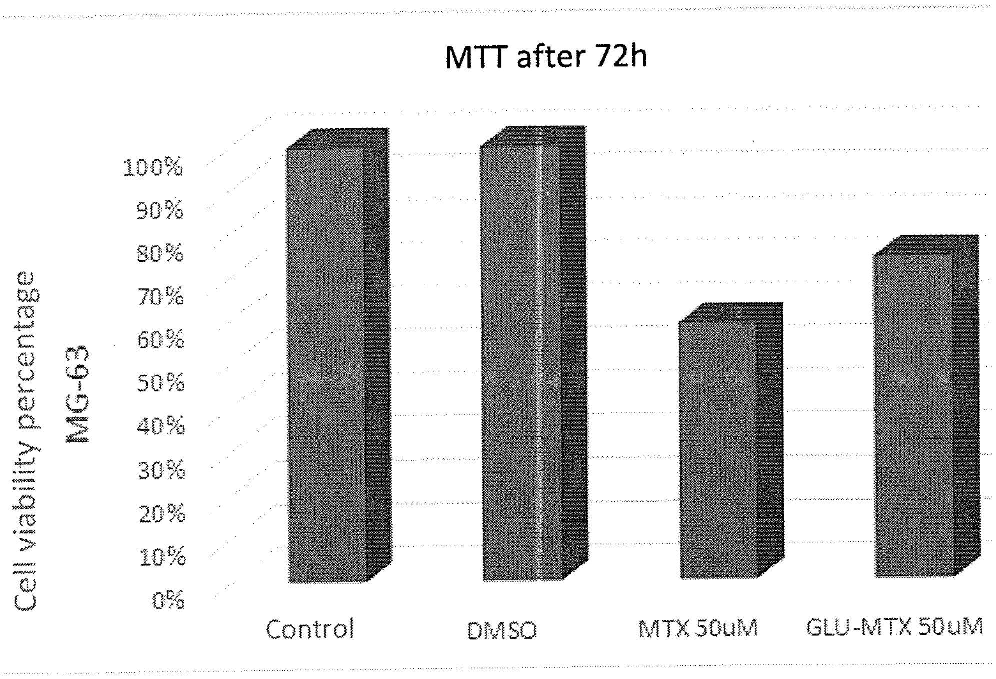


Figure 4

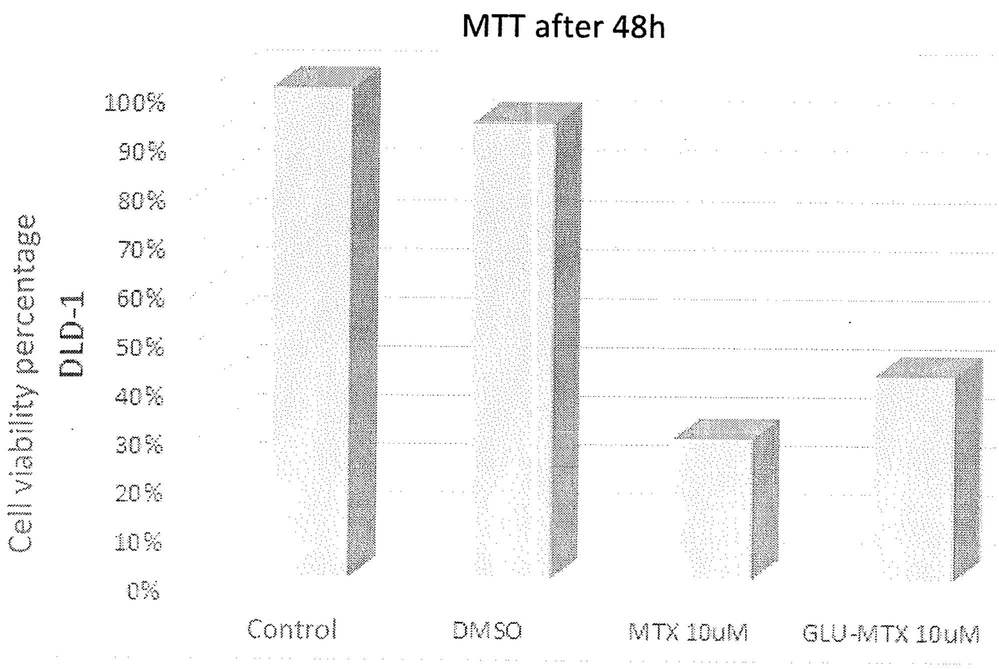


Figure 5

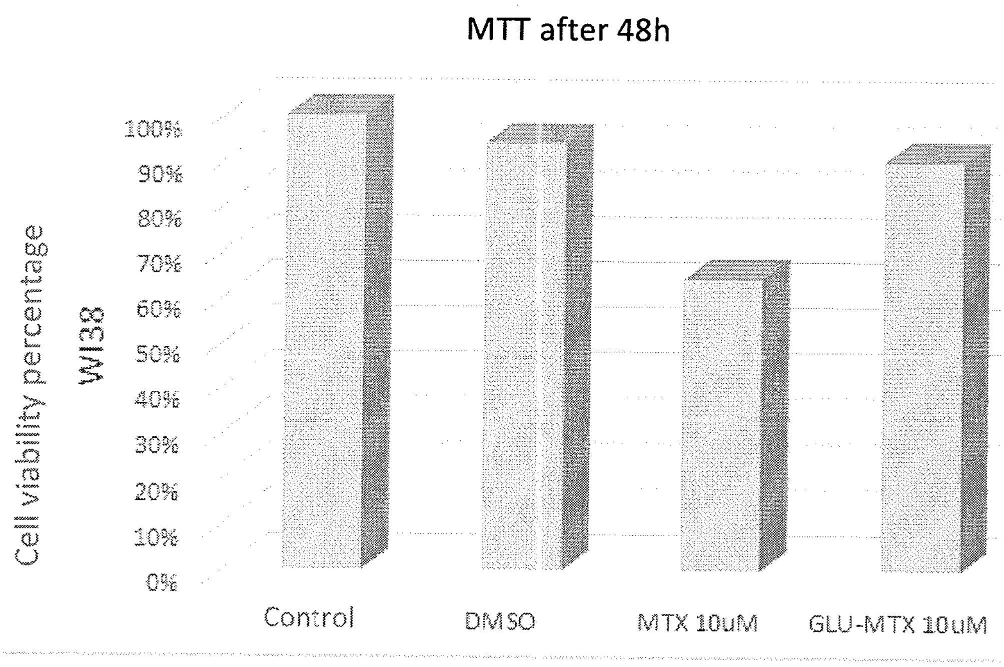


Figure 6

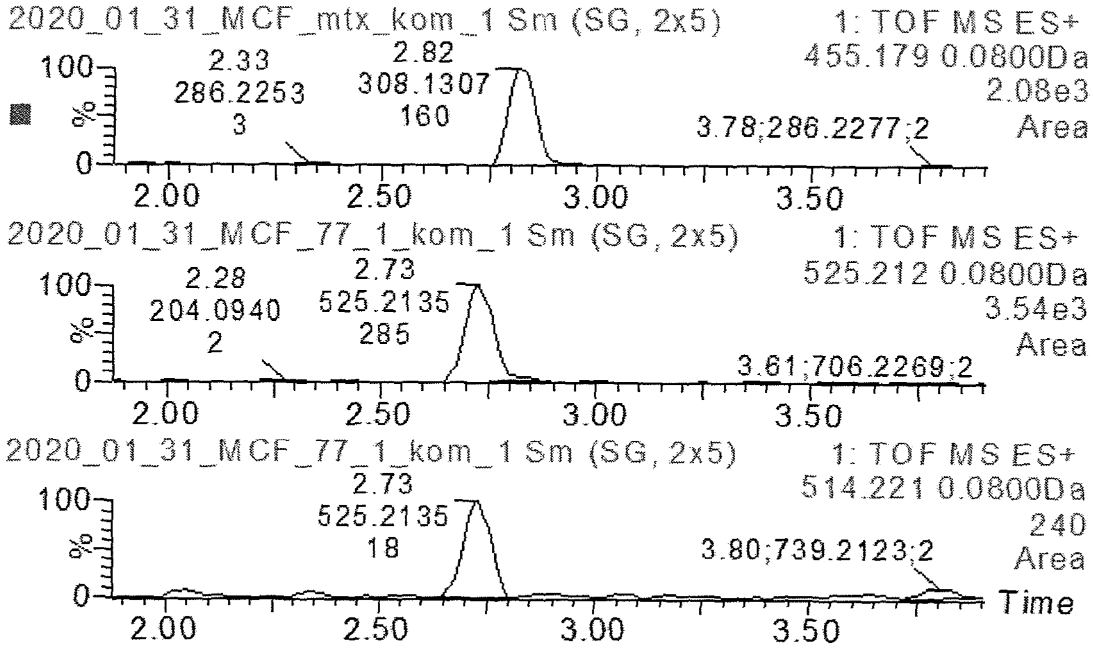


Figure 7

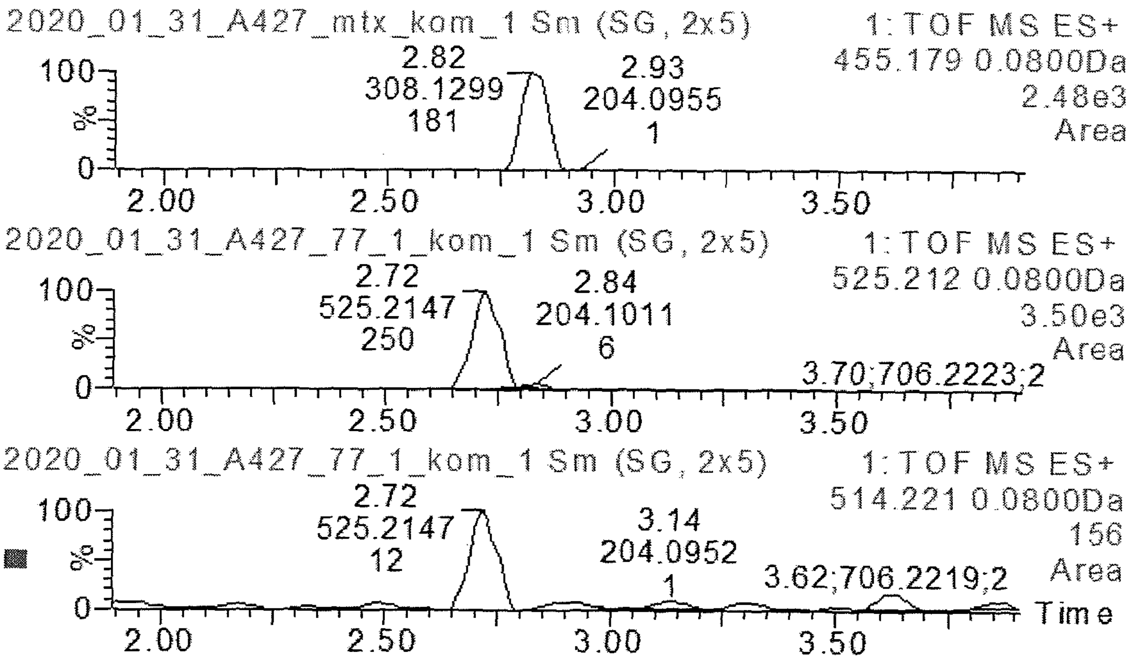


Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SRINIVASARAO M** ; **LOW PS.** Ligand-Targeted Drug Delivery.. *Chemical Reviews*, 2017, vol. 117 (19), 12133-64
- **CALVARESI EC** ; **HERGENROTHER PJ.** Glucose conjugation for the specific targeting and treatment of cancer.. *Chem Sci.*, 2013, vol. 4, 2319-33
- **L. ABDULRAHMAN** ; **O. BAKARE** ; **M. ABDULRAHMAN**. *The Chemical Approach of Methotrexate Targeting Frontiers in Biomedical Sciences*, 2016, vol. 1 (2), 50-73
- *CHEMICAL ABSTRACTS*, 59-05-2
- **MOURA JA** ; **VALDUGA CJ** ; **TAVARES ER** ; **KRETZER IF** ; **MARIA DA** ; **MARANHÃO RC**. Novel formulation of a methotrexate derivative with a lipid nanoemulsion.. *Int J Nanomedicine.*, 2011, vol. 6, 2285-2295